# FASCICULE DE BREVET EUROPEEN

(11) **EP 2 131 895 B1**
(45) Date de publication et mention de la délivrance du brevet: **26.12.2012**
(21) Numéro de dépôt: 08706388.9
(22) Date de dépôt: 05.03.2008
(51) Int. Cl.: A61M 5/142

(54) **POMPE DE NUTRITION ENTERALE PARENTERALE OU DE PERFUSION**
PERFUSIONS- ODER ENTERALE/PARENTERALE ERNÄHRUNGSPUMPE
PERFUSION OR ENTERAL/PARENTERAL FEEDING PUMP

(30) Priorité: 08.03.2007 EP 07405078
(43) Date de publication de la demande: 16.12.2009
(73) Titulaire: Rochat, Jean-Denis, 1272 Genolier (CH)
(72) Inventeur: Rochat, Jean-Denis, 1272 Genolier (CH)
(86) Numéro de dépôt international: PCT/CH2008/000090
(87) Numéro de publication internationale: WO 2008/106817

(56) Documents cités:
- WO-A-93/23096
- WO-A-2004/060435
- US-A1- 2005 191 194

## Description

La présente invention se rapporte à une pompe de nutrition entérale ou parentérale ou de perfusion à dispositif d'entraînement alimenté par batterie.

Les pompes actuellement sur le marché pour ce type d'utilisation ont une autonomie très limitée. Il s'agit pour l'essentiel de pompes péristaltiques dont le rendement global est particulièrement faible, généralement au maximum de l'ordre de 2%, voire sensiblement moins de 1%. Lorsqu'une telle pompe est alimentée par une batterie de l'ordre de 26'000J et travaille contre une pression de l'ordre de 2.10⁴Pa, elle n'a une autonomie que de l'ordre de 3 litres, ce qui correspond à 1,5 à 3 heures de fonctionnement suivant le débit. La recharge de l'accumulateur par adaptateur au réseau prend du temps et implique qu'une grande partie de l'utilisation de la pompe est en liaison électrique avec une prise murale, ce qui est un frein sérieux à la généralisation de l'usage des pompes en remplacement de la perfusion par goutte à goutte et gravité.

Les pompes péristaltiques ont l'inconvénient de souffrir, de manière intrinsèque, de grandes pertes d'énergie dues aux frottements qui réduisent fortement l'énergie utile au transfert dosé du liquide à perfuser. Les pompes à membranes utilisées ont de grands diamètres et travaillent avec de grandes courses, ce qui ne permet pas d'utiliser un moteur sans réducteur mécanique qui est lui aussi la source de pertes substantielles d'énergie.

Une pompe comprenant une membrane circulaire élastique et deux clapets est divulguée dans le document WO 93/23096 A. Le but de la présente invention est d'accroître de manière substantielle l'autonomie de pompage des pompes de nutrition entérale ou parentérale ou de perfusion en augmentant très sensiblement le rendement de la pompe, tout en assurant une sécurité optimale, ainsi qu'une grande précision.

A cet effet, la présente invention a pour objet une pompe de nutrition entérale ou parentérale ou de perfusion selon la revendication 1.

Non seulement l'autonomie d'une telle pompe est accrue de plusieurs dizaines de fois, mais cette pompe permet simultanément de réduire l'énergie nécessaire à son fonctionnement, rendant possible d'alimenter l'électroaimant moteur avec de simples batteries rechargeables standard du commerce de 1,2V de type AA ou LR6 suivant la norme utilisée, ce qui simplifie et réduit considérablement le travail et le coût de maintenance de ces pompes, de telles batteries de type standard se trouvant facilement sur le marché, notamment dans toute la grande distribution et à faible coût.

En outre le faible encombrement de ces pompes et de leur dispositif d'entraînement rend leur utilisation et leur installation aisée. Ce faible encombrement et sa grande autonomie en font une pompe parfaitement adaptée à une utilisation ambulatoire.

Le dessin annexé illustre, schématiquement et à titre d'exemple, une forme d'exécution de la pompe objet de la présente invention.
La figure 1 est une vue en élévation en coupe de cette forme d'exécution;
la figure 2 est un schéma montrant la courbe que forme le courant d'alimentation de l'électro-aimant;
la figure 3 est un schéma qui illustre un processus de traitement numérique de la courbe illustrée par la figure 2;
la figure 4 est un schéma montrant les courbes que forme le courant en cas d'occlusion en amont et en aval.

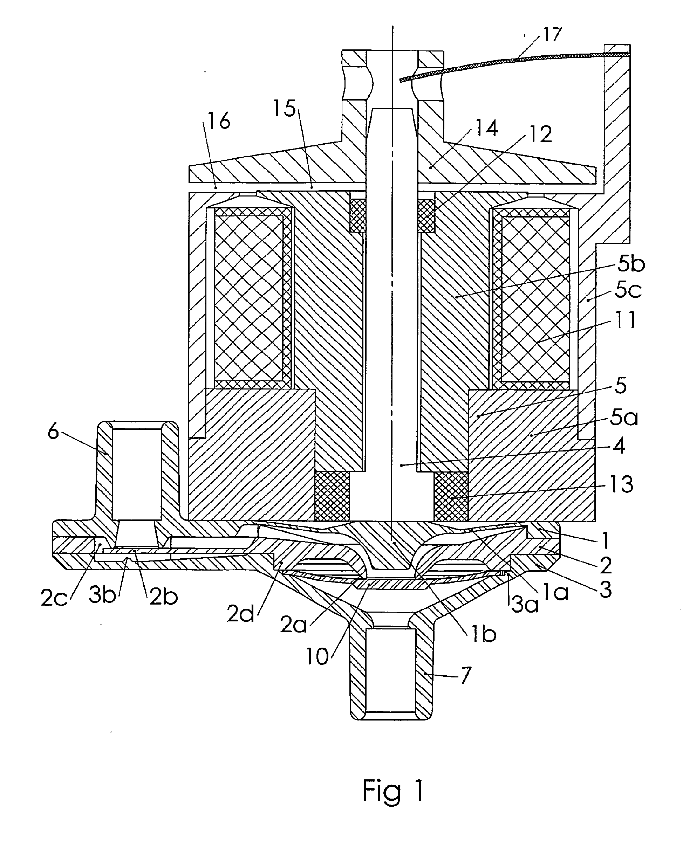

La figure 1 illustre une pompe, en particulier une pompe à usage unique, utilisée dans le domaine médical.

Comme on peut le constater, cette pompe à usage unique est essentiellement formée d'une enceinte en trois parties 1, 2, 3, deux parties 1, 3 formant la paroi de l'enceinte de pompage et une partie intermédiaire 2. Chacune des parties de paroi 1, 3 comporte un conduit d'admission 6 et un conduit de refoulement 7. La partie de paroi 1 présente une partie amincie formant une membrane annulaire 1a, entourant une partie d'actionnement épaisse 1b. La partie annulaire amincie la sert de membrane de pompage dont la partie centrale d'actionnement épaisse 1b sert à transmettre à la membrane annulaire la force exercée par un poussoir formé par le noyau mobile 4 d'un électroaimant 5 d'entraînement de la pompe. La déformation de la membrane 1a doit évidemment rester dans les limites de déformation élastique de la matière plastique formant la partie de paroi 1.

La partie intermédiaire 2 comporte une ouverture de communication 2a pour mettre en communication sélective les compartiments amont et aval de la pompe. Cette ouverture de communication 2a est disposée en regard de la partie centrale épaisse 1b de la membrane et un clapet de valve 2b est situé en regard d'une ouverture 2c vis-à-vis de l'extrémité interne du conduit d'admission 6 ménagé dans la partie de paroi 1. L'ouverture 2a se situe à l'extrémité d'une dépression, tandis que la partie centrale d'actionnement épaisse 1b forme une saillie qui s'engage dans l'ouverture 2a. La partie intermédiaire 2 comporte encore une saillie annulaire 2d tournée vers la partie de paroi 3, dont le rôle sera expliqué ci-après.

La partie de paroi 3 comporte un siège concentrique au conduit de refoulement 7, pour le positionnement d'un clapet 10 de contrôle de l'ouverture de communication 2a de la partie intermédiaire, qui fait également office de dispositif contre l'écoulement libre, dans le but d'empêcher tout écoulement de liquide lorsque l'usage unique n'est pas inséré dans la pompe. Ce clapet 10 est disposé entre cette ouverture de communication 2a et le conduit de refoulement 7 de la pompe. Pour éviter que du liquide puisse s'écouler par gravité à travers la pompe, le clapet 10 est appliqué contre l'ouverture 2a avec une pression de 4.10⁴Pa ± 1.10⁴Pa.

En position de repos, il ferme l'ouverture 2a et il est appliqué contre celle-ci dès que la différence de pression entre les côtés amont et aval de l'ouverture de communication 2a est inférieure à 4.10⁴Pa ± 1.10⁴Pa. Il s'écarte de cette ouverture 2a dès que la différence de pression sus-mentionnée est supérieure à 4.10⁴Pa ± 1.10⁴Pa.

La partie de paroi 3 présente un siège annulaire 3a pour le positionnement du clapet 10. Ce clapet 10 est retenu sur ce siège 3a par la saillie annulaire 2d de la partie intermédiaire 2. La partie de paroi 3 comporte encore une saillie 3b située en face du clapet 2b de contrôle du conduit d'admission 6, pour empêcher que ce clapet 2b ne vienne s'appliquer contre la face interne de la partie de paroi 3. Par cette disposition, la face du clapet 2b opposée à sa face adjacente à l'extrémité interne du conduit d'admission 6 de la pompe est exposée à la pression régnant dans le compartiment de la pompe situé en amont de l'ouverture de communication 2a de la partie intermédiaire 2. Ce clapet 2b peut ainsi fermer l'extrémité interne du conduit d'admission 6 en phase de refoulement de la pompe et l'ouvrir en phase d'aspiration.

Pour que la pompe décrite ci-dessus puisse fonctionner avec une grande autonomie, il est nécessaire de réunir un certain nombre de conditions, aussi bien en ce qui concerne la pompe proprement dite que son dispositif d'entraînement.

Tout d'abord, au niveau de la pompe proprement dite, la membrane 1a doit avoir un diamètre relativement petit compris entre 3 et 25mm, avantageusement situé autour de 16mm de manière à limiter sa force d'actionnement qui est le produit de la pression P par la surface S. Etant donné que l'on veut actionner cette membrane par un électroaimant de faible consommation, il faut aussi qu'il soit de faible dimension et que la course de la membrane 1a, entraînée par le noyau-poussoir 4 de l'électroaimant, se situe entre 0,2 et 2mm, avantageusement de l'ordre de 0,5mm. Dans ces conditions, la course de la membrane 1a permet son entraînement direct par le noyau-piston 4 de l'électroaimant et évite tout réducteur mécanique qui diminue sensiblement le rendement global de la pompe.

Avantageusement, l'épaisseur de la membrane élastique la se situe entre 0,1 et 0,7mm, de préférence de l'ordre de 0,3mm. Ces dimensions permettent d'utiliser le même matériau thermoplastique pour la membrane 1a que pour la partie 1 de paroi de l'enceinte de la pompe, ce qui permet de réaliser la partie 1 et la membrane 1a en une seule opération d'injection. Parmi les matériaux thermoplastiques-utilisables, on peut citer le PC, le PVC, L'ABS, le PP ou le PE notamment. Le choix est fonction du coût, de la précision et de la stabilité des caractéristiques élastiques après stérilisation et stockage sur une durée maximum de trois ans. Le PC est le matériau qui correspond le mieux à ce cahier des charges.

L'électroaimant 5 qui constitue la partie motrice réutilisable de la pompe est un électro-aimant à pot cylindrique alimenté par une tension de 5V. Il faut donc un élévateur de tension entre la batterie de 1,2V et l'alimentation de 5V de l'électroaimant. Son circuit magnétique comporte deux entrefers, un entrefer interne 15 et un entrefer externe 16 de part et d'autre du logement de la bobine 11. Pour que le frottement soit le plus réduit possible, le noyau-piston en métal dur 4 formant la partie mobile de l'électroaimant 5 est guidé dans deux paliers 12, 13 en céramique lubrifiante. Le noyau-piston 4 est solidaire de l'armature mobile 14 de l'électroaimant à surface de réluctance Sp pratiquement constante. Comme on peut le constater sur la figure 1, pour réduire les réluctances des entrefers 15, 16 et augmenter au maximum la force qu'ils engendrent, ils recouvrent tous deux presque complètement le logement de la bobine 11, ce qui nécessite de réaliser le pot cylindrique de l'électro-aimant 5 en trois pièces, 5a, 5b, 5c.

Le pot de l'électro-aimant 5 a un diamètre compris entre 20 et 40mm, typiquement 24mm et une hauteur comprise entre 15 et 25mm, typiquement 20mm. Le taux de remplissage du logement du pot de l'électroaimant 5 par la bobine 11 est de l'ordre de 80%. Cette bobine 11 a un diamètre externe de 18 à 24mm, typiquement 21mm et une hauteur de 9 à 14mm, typiquement de 11mm. La surface de réluctance Sp de l'armature mobile 14 se situe entre 50 et 120mm², typiquement 70mm². Les entrefers intérieur 15 et extérieur 16 ont des valeurs comprises entre 0,2 et 2mm, de préférence de l'ordre de 0,5mm. Dans une forme préférée, l'entrefer intérieur 15 est de 0,5mm et l'entrefer extérieur est de 0,6mm.

L'obtention d'un temps de réaction de l'électroaimant apte à être en accord avec les temps de transfert du liquide pompé (typiquement 35ms) donnant un rendement maximum, nécessite une adaptation d'impédance entre la source et la charge.

Pour économiser du courant, on fait en sorte que la membrane 1a revienne d'elle-même et suffisamment rapidement dans sa position de repos. A cet effet, il faut qu'en position de repos, cette membrane 1a soit soumise à une précontrainte de pression comprise entre 1.10⁴ et 4.10⁴Pa, typiquement de 2.10⁴Pa.

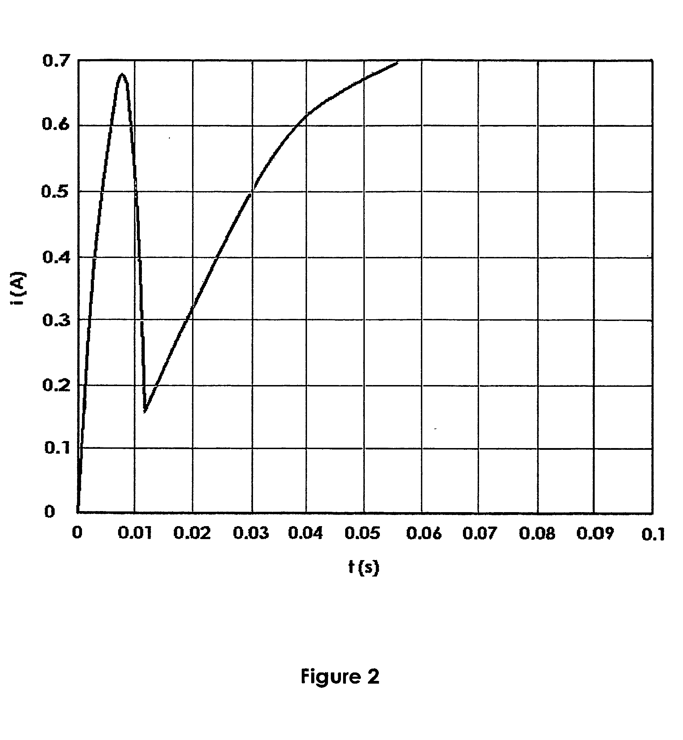

Etant donné que l'électroaimant ne sert qu'à pousser la membrane 1a, celle-ci revenant d'elle-même à sa position de repos, le dispositif d'entraînement comporte des moyens d'analyse de la courbe décrite par le courant d'alimentation de la bobine 11. Cette courbe est illustrée par la figure 2 et montre que le courant passe par un maximum, puis par un minimum, correspondant à la fermeture des entrefers 15, 16. Le courant croît ensuite pour atteindre la valeur de I=U/R où R est la valeur de la résistance de la bobine 11.

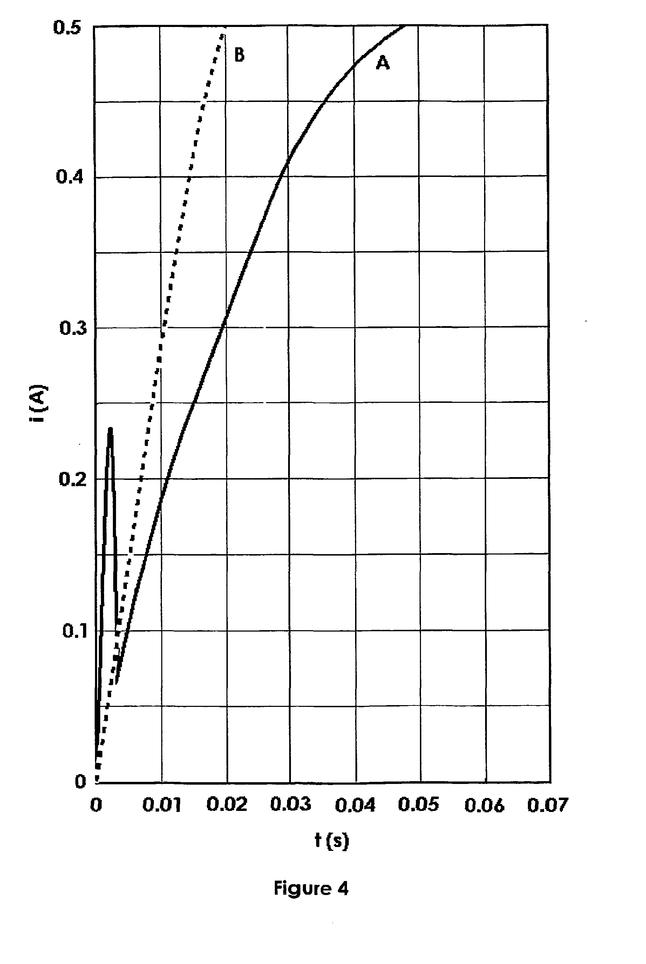

Pour économiser l'énergie, le dispositif d'alimentation de l'électroaimant 5 comporte des moyens pour analyser la courbe de la figure 2 en vue de détecter le passage de la, courbe du courant I à un minimum et d'interrompre alors, à chaque cycle de pompage, la tension d'alimentation de l'électroaimant 5. Par la même analyse, on peut aussi détecter une occlusion en amont, qui se distingue par le fait que la membrane ne reprend pas sa position de repos et le piston remonte alors au moyen d'un très faible ressort 17 ne servant qu'à permettre la remontée du piston (le ressort 17 doit vaincre les forces de frottement du guidage et le poids du piston, ce qui équivaut à une force totale d'environ 0.08 N), ou en aval du conduit, comme illustré par les courbes A, respectivement B de la figure 4 et déclencher dans ce cas une alarme.

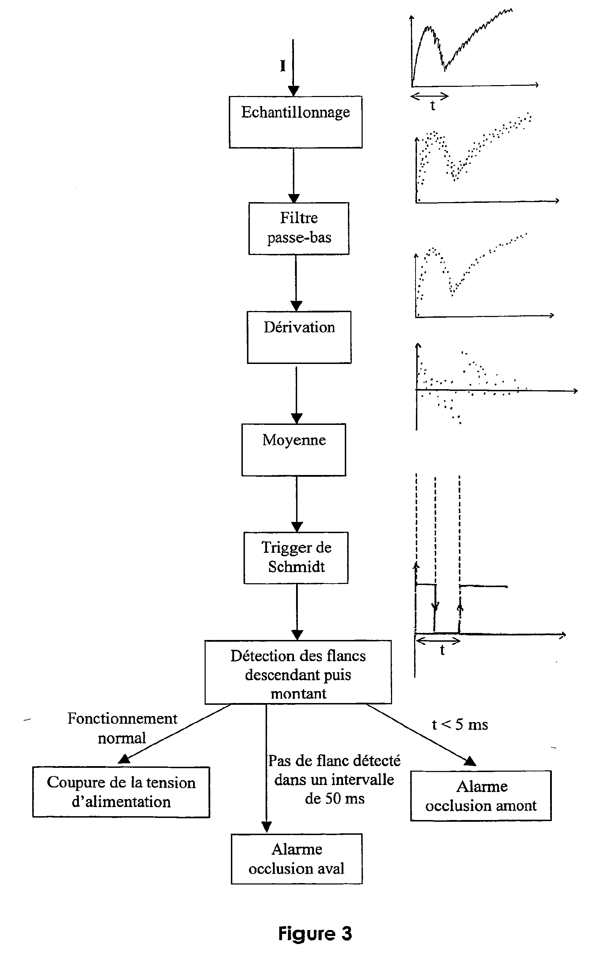

Dans l'exemple illustré par la figure 3, le signal est traité de façon numérique, mais on pourrait aussi le traiter de façon analogique.

Le schéma de la figure 3 illustre les différentes étapes de traitement du signal avec l'évolution du signal traité à chaque étape. On constate qu'à la fin du traitement, on détecte la présence ou non de flancs descendant puis montant à la fin de la durée t normale de déplacement de la membrane 1a correspondant à la demi-période du cycle de pompage. Si ces flancs sont détectés, le fonctionnement est normal et la tension d'alimentation est coupée jusqu'à la fin du cycle de pompage, comme décrit précédemment. Ce mode de détection assure donc que l'électroaimant est allé au bout de sa course, garantissant ainsi la précision de chaque volume pompé.

En admettant, dans cet exemple, que la pompe travaille à une fréquence maximum de 10Hz, si au bout de 50ms aucun flan n'a été détecté, ceci signifie que la membrane 1a n'a pas pu être déplacée en raison d'une occlusion aval (courbe B de la figure 4) et le dispositif de détection déclenche l'alarme d'occlusion aval. Si le dispositif de détection détecte un flanc descendant dans un intervalle t < 0,5ms, ceci signifie qu'il y a une occlusion amont (courbe A de la figure 4) et le dispositif de détection déclenche l'alarme d'occlusion amont.

Dans l'exemple décrit précédemment, le volume pompé par cycle de pompage est de 0,058ml, la fréquence maximum de pompage se situe entre 8 et 12Hz, typiquement 10Hz. Ceci correspond à des débits maximum qui se situent entre 1,5 et 2,5 l/h, typiquement de 2 litres avec une précision de l'ordre de 5%.

Avec un accumulateur de 7000 à 10'000J, typiquement de 8600J, contre une pression de 2.10⁴Pa, la pompe objet de la présente invention peut avoir une autonomie de 10 à 120 litres, typiquement de 100 litres. Dans les mêmes conditions, une pompe péristaltique avec un accumulateur de 26'000J a une autonomie de 3 litres. Ceci permet de constater l'énorme progrès que la présente invention permet de réaliser dans le domaine des pompes de nutrition entérale et parentérale et de perfusion.

Une comparaison entre les rendements des pompes péristaltiques utilisées dans les domaines de la nutrition en particulier avec la pompe objet de la présente invention a permis de constater que les rendements sont multipliés pratiquement par 100 dans le cas de la présente invention.

## Revendications

1. Pompe de nutrition entérale ou parentérale ou de perfusion à dispositif d'entraînement alimenté par batterie, comprenant une membrane circulaire élastique (1a) et deux clapets (2b, 10) situés respectivement en amont et en aval de cette membrane (1a), laquelle a un diamètre de 3 à 25mm et est soumise à une pré-contrainte de pression comprise entre 1.10⁴ et 4.10⁴Pa apte à lui permettre de revenir à sa position de repos à une fréquence de travaille maximum comprise entre 8 et 12Hz avec une course comprise entre 0,2 et 2mm, correspondant à un débit maximum de 1,5 à 2,5 l/h et **caractérisé en ce que** le dispositif d'entraînement comprend un électroaimant (5) dont la partie mobile est en liaison directe avec ladite membrane (1a), une batterie d'alimentation standard de cet électroaimant dont l'énergie disponible est de 7000 à 10'000J, des moyens pour analyser le courant d'alimentation de l'électro-aimant et détecter à chaque cycle d'entraînement de pompage une intensité minimum déterminée du courant d'alimentation correspondant à la fermeture de l'entrefer de l'électro-aimant (5) et des moyens pour interrompre le courant d'alimentation une fois cette intensité minimum atteinte, afin d'assurer une autonomie de pompage comprise entre 10 et 120 litres.

2. Pompe selon la revendication 1, dans laquelle la membrane circulaire (1a) a un diamètre compris entre 12 et 20mm, la précontrainte de pression est de l'ordre de 2.10⁴Pa, la fréquence de travail maximum est de l'ordre de 10Hz, la course de la membrane (1a) est comprise entre 0,4 et 0,8mm, le débit maximum est de 2 l/h, l'énergie de la batterie d'alimentation de l'électroaimant est de 8000 à 9000J, permettant une autonomie de la pompe de 80 à 100 litres.

3. Pompe selon l'une des revendications précédentes, dans laquelle le clapet (10) situé en aval de la membrane (1a) est appliqué contre son siège (2a) en position de repos avec une pression de 4.10⁴Pa ± 1.10⁴Pa.

4. Pompe selon l'une des revendications précédentes, comportant des moyens pour analyser l'évolution du courant d'alimentation de l'électroaimant et pour détecter une évolution caractéristique d'une occlusion en amont ou en aval de la pompe, ces moyens pour analyser l'évolution du courant étant reliés à un dispositif d'alarme pour l'activer lorsqu'ils détectent que la courbe du courant d'alimentation est caractéristique d'une occlusion en amont ou en aval de la pompe.

5. Pompe selon l'une des revendications précédentes, dans laquelle l'électro-aimant (5) travaille avec une tension de 5V, il s'agit d'un électro-aimant à pot cylindrique à deux entrefers (15, 16), intérieur et extérieur < 1mm, recouvrant partiellement la bobine (11) de diamètre compris entre 20 et 40mm et de hauteur comprise entre 12 et 20mm, à taux de remplissage de l'ordre de 80% et dont la partie mobile (14) présente une surface de réluctance Sp à peu près constante.

6. Pompe selon l'une des revendications précédentes, dans laquelle la batterie d'alimentation est un accumulateur rechargeable de 1,2V et de 8600J ou plus.

7. Pompe selon l'une des revendications précédentes, dans laquelle le noyau-piston en métal dur(4) de l'électro-aimant (5) est guidé dans deux paliers 12, 13 en céramique auto-lubrifiée.

8. Pompe selon la revendication 4 dans laquelle la partie mobile (4) de l'électroaimant (5) est associée à un ressort (17) dont la force est calculée pour vaincre les forces de frottement et le poids de la partie mobile (4) de l'électroaimant (5) pour ramener cette partie mobile (4) en position de repos dans le cas d'une occlusion amont.

## Claims

1. Enteral, parenteral or infusion feeding pump with a battery-powered drive mechanism, comprising a resilient circular membrane (1a) and two valves (2b, 10) situated upstream and downstream, respectively, of this membrane (1a), which has a diameter of 3 to 25 mm and is subjected to a pressure preload of between 1.10⁴ and 4.10⁴ Pa so as to enable it to return to its rest position at a maximum working frequency of between 8 and 12 Hz with a stroke of between 0.2 and 2 mm, corresponding to a maximum flow rate of 1.5 to 2.5 Uh and **characterised in that** the drive mechanism comprises an electromagnet (5) of which the moving part is directly connected to said membrane (1a), a standard battery to power this electromagnet with an available energy of 7000 to 10,000 J, means for analysing the electromagnet supply current and detecting in each pump drive cycle a predetermined minimum value of the supply current corresponding to closure of the air gap of the electromagnet (5), and means for interrupting the supply current once this minimum value is reached, in order to ensure that one battery charge cycle can pump between 10 and 120 litres.

2. Pump according to claim 1, wherein the circular membrane (1a) has a diameter of between 12 and 20 mm, the pressure preload is around 2.10⁴ Pa, the maximum working frequency is around 10 Hz, the stroke of the membrane (1a) is between 0.4 and 0.8 mm, the maximum flow rate is 2 Uh, and the energy of the battery powering the electromagnet is from 8000 to 9000 J, allowing one battery charge cycle to pump from 80 to 100 litres.

3. Pump according to any of the preceding claims, wherein the valve (10) situated downstream of the membrane (1a) is pressed against its seat (2a) in the rest position with a pressure of 4.10⁴ Pa ± 1.10⁴ Pa.

4. Pump according to any of the preceding claims, comprising means for analysing the variation of the electromagnet supply current and detecting a variation characteristic of a blockage upstream or downstream of the pump, these means for analysing the variation of the current being connected to an alarm device to activate it when they detect that the curve of the supply current is characteristic of a blockage upstream or downstream of the pump.

5. Pump according to any of the preceding claims, wherein the electromagnet (5) operates at a voltage of 5 V and has a cylindrical pot with two air gaps (15, 16), an inner air gap and an outer air gap <1 mm, partially covering the coil (11) of which the diameter is between 20 and 40 mm and of which the height is between 12 and 20 mm, with a fill factor of around 80%, and of which the moving part (14) has an approximately constant area of reluctance Sp.

6. Pump according to any of the preceding claims, wherein the power supply battery is a 1.2 V rechargeable battery holding 8600 J or more.

7. Pump according to any of the preceding claims, wherein the hard metal piston core (4) of the electromagnet (5) is guided by two self-lubricating ceramic bearings (12, 13).

8. Pump according to claim 4, wherein the moving part (4) of the electromagnet (5) is provided with a spring (17) of which the force is calculated to overcome the forces of friction and the weight of the moving part (4) of the electromagnet (5) in order to return this moving part (4) to the rest position in the event of an upstream blockage.

## Patentansprüche

1. Perfusions- oder enterale oder parenterale Ernährungspumpe mit einer von einer Batterie versorgten Antriebseinrichtung, die eine kreisförmige elastische Membran (1a) und zwei Ventile (2b, 10) aufweist, die dieser Membran (1a) vorgeschaltet bzw. nachgeschaltet gelegen sind, wobei die Membran einen Durchmesser von 3 bis 25 mm hat und einer Druckvorspannung von 1.10⁴ bis 4.10⁴ Pa unterworfen ist, die geeignet ist, sie in ihre Ruheposition zurückkehren zu lassen bei einer maximalen Arbeitsfrequenz von 0,2 bis 2 mm entsprechend einem maximalen Durchsatz von 1,5 bis 2,5 l/h, **dadurch gekennzeichnet, dass** die Antriebseinrichtung einen Elektromagneten (5) aufweist, dessen beweglicher Teil in direkter Verbindung mit der Membran (1a) ist, eine Standard-Versorgungsbatterie für den Elektromagneten, dessen verfügbare Energie zwischen 7.000 bis 10.000 J beträgt, Mittel zum Analysieren des Versorgungsstroms des Elektromagneten und zum Detektieren bei jedem Pumpenarbeitszyklus einer Minimalintensität, die vom Versorgungsstrom bestimmt ist, der einem Schließen eines Spalts des Elektromagneten (5) entspricht und Mittel zum Unterbrechen des Versorgungsstroms, sobald dieses Intensitätsminimum erreicht ist, um eine Pumpenbetriebsdauer zwischen 10 und 120 Liter zu gewährleisten.

2. Pumpe nach Anspruch 1, wobei die kreisförmige Membran (1a) einen Durchmesser von 12 bis 20 mm hat, die Druckvorspannung in der Größenordnung von 2.10⁴ Pa liegt, die maximale Arbeitsfrequenz in der Größenordnung von 10 Hz liegt, der Hub der Membran (1a) von 0,4 bis 0,8 mm beträgt, der maximale Durchsatz bei 2 l/h liegt, die Energie der Versorgungsbatterie des Elektromagneten 8.000 bis 9.000 J beträgt, was eine Pumpenbetriebsdauer von 80 bis 100 l gestattet.

3. Pumpe nach einem der vorhergehenden Ansprüche, wobei die das Ventil (10), das der Membran nachgeschaltet gelegen ist, gegen seinen Sitz (2a) in Ruheposition mit einem Druck von 4.10⁴ Pa ± 1.10⁴ Pa anliegt.

4. Pumpe nach einem der vorhergehenden Ansprüche, wobei sie Mittel zum Analysieren des Versorgungsstromverlaufs des Elektromagneten und zum Detektieren eines charakteristischen Okklusionsverlaufs vor oder nach der Pumpe aufweist, wobei die Mittel zum Analysieren des Stromverlaufs mit einer Alarmeinrichtung verbunden sind, um sie zu aktivieren, sobald sie detektieren, dass die Versorgungsstromkurve direkt charakteristisch für eine Okklusion vor oder nach der Pumpe ist.

5. Pumpe nach einem der vorhergehenden Ansprüche, wobei der Elektromagnet (5) mit einer Spannung von 5 V arbeitet, es sich um einen zylinderförmigen Elektromagneten bei zwei Spalten (15, 16) handelt, innerer und äußerer < 1mm, die teilweise die Spule (11) bei einem Durchmesser von 20 bis 40 mm und einer Höhe von 12 bis 20 mm abdecken, bei einem Füllgehalt in der Größenordnung von 80% und dessen beweglicher Teil (14) eine ungefähr konstante Reluktanzoberfläche Sp darstellt.

6. Pumpe nach einem der vorhergehenden Ansprüche, wobei die Versorungsbatterie ein wiederaufladbarer Akkumulator von 1,2 V und von 8.600 J und mehr ist.

7. Pumpe nach einem der vorhergehenden Ansprüche, wobei der Kernkolben (4) aus Hartmetall des Elektromagneten (5) in zwei selbstschmierenden Lagern (12, 13) aus Keramik geführt ist.

8. Pumpe nach Anspruch 4, wobei der bewegliche Teil (4) des Elektromagneten einer Feder (17) zugeordnet ist, deren Kraft berechnet ist, um die Reibkräfte und das Gewicht des beweglichen Teils (4) des Elektromagneten (5) zu überwinden, um das bewegliche Teil in eine Ruheposition im Falle einer vorgeschalteten Okklusion zurückzuführen.
